# EUROPEAN PATENT APPLICATION

(11) **EP 4 667 100 A1**
(43) Date of publication of application: **24.12.2025**
(21) Application number: 24185441.3
(22) Date of filing: 28.06.2024
(51) Int. Cl.: B01L 3/00

(54) **TESTING DEVICE**

(30) Priority: 19.06.2024 CN 202410795981
(71) Applicant: Zhejiang Orient Gene Biotech Co., Ltd, Anji Huzhou Zhejiang 313300 (CN)
(72) Inventor: Feng, Haiying, Huzhou, Zhejiang, 313300 (CN); Pan, Lijuan, Huzhou, Zhejiang, 313300 (CN); Fang, Jianqiu, Huzhou, Zhejiang, 313300 (CN)
(74) Representative: Petraz, Gilberto Luigi

(57) **Abstract**

The invention provides a testing device, and the testing device includes: a first absorbent material for contacting a liquid sample, wherein a first test area is arranged on the first absorbent material; and a second absorbent material for contacting the liquid sample, wherein a second test area is arranged on the second absorbent material. In a preferred embodiment, a flow rate of the liquid sample on the first absorbent material is smaller than a flow rate of the liquid sample on the second absorbent material.

## Description

### CROSS-REFERENCE TO RELATED APPLICATIONS

This application claims priority to the Chinese Patent Application, Application No. CN2024107959817, filed on June 19, 2024, and all disclosures of the Applications, including but not limited to the specification, abstract, claims and accompanying drawings of this application are hereby made a part of this application.

### BACKGROUND OF THE INVENTION

### Field of the Invention

The invention relates to the field of in vitro test, and in particular, to a testing device for testing an analyte in a liquid sample and the biological property of the liquid sample by lateral flow.

### Description of the Related Art

The following description is merely an introduction of some background knowledge and does not constitute any limitation to the invention.

The composition and physical characteristics of liquid are often used in test to understand the situation of the source of the liquid. For example, oxidants for testing biological liquids, pH value, specific gravity, creatinine, bilirubin, glucose, and other similar indicators can show health status or contamination. Food and beverage test items include pH value, specific gravity, contamination by bacteria or toxic substances, and the like. Test indicators of soil and water samples also include pH value, specific gravity, bacteria, lead or mercury contamination.

Generally, these rapid chemical tests can be accomplished by using test strips with a series of chemical test pads directly dipped with samples and reading test results directly, without using capillary action. The test pad chemically reacts with the test sample or sample components to cause a color change. Usually, an operator only needs to immerse the test strips in the liquid, then observe the color change of the test pad, and compare the color of the test pad with that of the result card to determine the test result.

However, such test strips of "dipping samples - reading results" has many disadvantages, and especially they cannot be combined with modern new rapid immunoassay devices. Nevertheless, these chemical tests are still necessary. The new rapid immunoassay devices are designed to reduce the contact opportunity between the operator and the samples under test. By using the testing method of "dipping samples - reading results", the operator must open the testing device, which will cause the samples to be contaminated by the operator or other reagents on the test strip.

For example, U.S. Pat. No. US7, 595, 196 mainly discloses a test strip, and a hydrophobic medium is arranged on the test strip and can prevent the liquid from flowing back from the test pad to an absorbent pad, thus causing the reverse flow of the liquid sample. Although this can be improved, in the existing traditional products, there is still the phenomenon that colored substances on reaction pads diffuse with the forward flow of the liquid. In addition, liquids among different test pads are mixed, which causes the interference of the test pads.

Therefore, improved methods and equipment to test adulteration of samples are very necessary.

### BRIEF SUMMARY OF THE INVENTION

For the disadvantages of the above conventional technology, the invention provides a lateral flow testing device or a test strip, such that a liquid sample independently contacts with various test areas; or a liquid sample is divided into multiple independent running routes, such that the liquid sample independently contacts with various test areas, which reduces the interference caused by a fact that liquids among the various test areas are mixed due to the flow of a single liquid. In particular, this can avoid the diffusion of colored substances on the test area.

Therefore, the invention provides a rapid chemical testing device for liquid samples and a use method thereof. In a specific embodiment, the invention provides a lateral flow test strip for detecting physical properties of liquid samples. The device and the method are applicable in various forms, and can be used to detect whether liquids or liquefied samples are contaminated or adulterated. For example, the device can detect pH value, specific gravity, or whether subjects try to cover up illegal drug use and adulterate urine collection samples. For another example, the device and method can also be used to detect whether food samples are contaminated by bacteria, or whether lead or other undesirable substances exist in foods and beverages.

A first aspect of the invention provides a device for analyzing a liquid sample. The test strip included in the device includes a first absorbent material for carrying the liquid sample through the test strip; a second absorbent material for carrying the liquid sample through the test strip, or both the first absorbent material and the second absorbent material can absorb the liquid sample and allow the liquid sample to flow thereon. In some embodiments, a first test area is arranged on the first absorbent material, and a second test area is arranged on the second absorbent material, and the first test area and the second test area independently receive the liquids, such as liquid samples, flowing on the absorbent materials. In some embodiments, the first test area contacts the liquid sample on the first absorbent material, and then the second test area receives or contacts the liquid sample on the second absorbent material. Alternatively, in some embodiments, the first test area is located upstream and the second test area is located downstream, such that the first test area contacts the liquid sample and then the second test area contacts the liquid sample. However, the liquid samples contacted by the two test areas are from two different fluid paths, instead of being from a same fluid path. Therefore, respective reactions are made on respective test areas on the two fluid paths, such that the second test area does not substantially contact the liquid sample from the first absorbent material, thus avoiding the interference of the reaction caused by the flow of the liquid between the test areas. Similarly, the first test area mainly receives the liquid sample on the first absorbent material rather than the liquid sample on the second test area.

In some embodiments, the first absorbent material covers the second absorbent material, but there is an impermeable membrane between the first absorbent material and the second absorbent material, which ensures that the two fluid flow paths will not interfere with each other. In some embodiments, the first absorbent material has a first end and a second end opposite to the first end, and the second absorbent material also has a first end and a second end. The first end of the first absorbent material is overlapped with the first end of the second absorbent material to contact the liquid sample, and the liquid sample is allowed to be transported on the first absorbent material and the second absorbent material.

In some embodiments, when the first absorbent material covers the second absorbent material, there is no permeable membrane between them. When the first absorbent material and the second absorbent material contact the liquid, a flow rate of the liquid sample on the second absorbent material can be greater than a flow rate of the liquid sample on the second absorbent material, and liquid flow can be formed between the two absorbent materials. However, when the liquid absorbed by the second absorbent material flows below the first test area in advance, the liquid sample on the second absorbent material flows to the first test area through part of the first absorbent material; and when the liquid sample on the first absorbent material flows to the first test area, the convergence of fluids from two different fluid paths can slow down the liquid sample on the first absorbent material to continue forward flow, thus effectively preventing a reagent on the first test area from forward diffusion. In addition, a flow direction of the liquid sample on the second absorbent material to the first test area is from bottom to top and similar to a direction perpendicular to a test pad; and a flow direction of the liquid sample on the first absorbent material is from left to right and similar to a direction parallel to the test pad. In a flow process, part of the liquid flows to the first test area (most of the flow direction is not perpendicular, but has an inclined angle with the test pad), and the other of the liquid continues to flow forward. If there is a lateral liquid flow (not parallel to the flow of the liquid sample on the absorbent material but perpendicular to the flow of the liquid) to prevent the liquid from flowing forward or slow down the flow rate of the liquid, the reagent on the first test area can be prevented from diffusing forward or backward or diffusing downstream along the flow path of the first absorbent material. The diffusion will cause the reagent to flow to the downstream of the test area, such as the second test area, thus causing interference in the results. A special example is that an alkaline chemical reagent is tested on the first test area while the pH value of the sample is tested on the second test area. If alkaline substances flow to the second test area with the flow of the liquid, the sample becomes alkaline, resulting in false positive or false negative results. Therefore, the test results are inaccurate. For example, urine is originally acidic (for example, pH=5), but the interference of alkaline substances changes the acidity and alkalinity of the sample, resulting in false negative (for example, pH=8).

In some embodiments, in order to overcome the interference caused by the flow of the liquid between the first test area and the second test area, the first absorbent material can be made shorter than the second absorbent material, and the first test area is located at the second end of the first absorbent material, while the second test area is located at the downstream of the first test area, but on the second absorbent material. Therefore, when the liquid contacts the ends of the first absorbent material and the second absorbent material and the liquid flowing through the first absorbent material flows to the second end, it will directly flow to the first test area instead of forward flow. Since the liquid sample has reached the end of the first absorbent material, it will not flow forward; the liquid sample flowing on the second absorbent material can continue to flow beyond the end of the first absorbent material and contact the second test area located at the downstream of the first test area; therefore, the second test area is mainly used to contact the liquid sample flowing on the second absorbent material, thereby reducing the liquid from the first absorbent material and avoiding the flow of the liquid between the test areas; and each test area is allowed to accomplish the test and receive the liquid from different paths relatively independently. In other words, in a same liquid flow path, only one test area is arranged to test the property of an analyte or a liquid, and the test area is provided at most two, while the test area for testing an analyte in another liquid sample is arranged on the second fluid path on the second absorbent material. Of course, the first fluid path and the second fluid path can be isolated by the impermeable membrane or have different flow rates; in an optional embodiment, the flow rate of the liquid sample on the second absorbent material is greater than the flow rate of the liquid sample on the first absorbent material.

In some embodiments, it is hoped that the flow rate of the liquid is relatively fast, such that the reaction can be completed as quickly as possible; however, one disadvantage of the fast flow rate is that it is easy to cause the reagents on the test area to be dissolved in the liquid and continue the forward flow. Therefore, in some embodiments, one third absorbent material is arranged between the first absorbent material and the first test area. The absorbent material is used to guide the liquid to flow from the first absorbent material to the first test area, and plays a role in slowing down the flow rate of the liquid; in addition, it can change the flow direction of the liquid. When the flow rate of the liquid sample on the first absorbent material is very fast, the third absorbent material can be used to slow down the flow rate and the liquid is allowed to flow to the first test area, thus reducing the impact of the excessively fast flow rate on the first test area. In addition, due to the provision of the third absorbent material, the liquid originally flows forward, and when it meets the third absorbent material, the liquid flows from bottom to top (perpendicular to the test pad), which slows down the flow rate of the liquid and is more conducive to the liquid flowing to the first test area. Therefore, in some embodiments, the flow rate of the liquid sample on the third absorbent material is lower than the flow rate of the liquid sample on the first absorbent material. For example, the absorbent materials with a dense texture or a large unit volume are selected to slow down the flow rate of the liquid. For example, those sparse materials with a large pore size are used as the first absorbent material, absorb water quickly, and can be filled quickly with the water. However, when encountering the dense third absorbent material, the liquid flows to the first test area through the third absorbent material, and the test area is filled slowly with the liquid sample, thereby reducing the impact of the liquid sample on the test area.

A fundamental reason is that chemical substances are treated in the test area of the invention, and the material of the test area is also the absorbent material. These meta-materials are made into chemical test pads or reaction pads by soaking in a chemical solution, filling the chemical solution with the absorbent material, and then drying. In this case, when the liquid sample is wet, it is hoped that all the chemical reagents of the chemical reagent pad can participate in the performance test of the analyte in the liquid sample or the liquid sample. If the flow of the liquid causes the reagents in the test area to flow back to the first absorbent material, the continued flow of the absorbent material interferes with the reagents in the test areas located downstream. Another important reason is that when these chemical reagents react, new colored substances are generally produced, and precipitate or adhere to the absorbent pad. If the liquid continues to flow downstream and the colored substances flow back to the first absorbent material with the flow of the liquid, this also allows the colored substances to flow to the test area located downstream and causes interference. Therefore, when one third absorbent material is arranged between the first absorbent material and the test pad containing the chemical reagents, the flow rate of the liquid changes (the flow rate of the liquid is reduced), and the flow direction of the liquid changes, such that the first test area can better complete the reaction independently, and the diffusion of substances on the first test area with the flow of the liquid is reduced.

In some specific embodiments of the invention, the device further includes a support located under the second absorbent material. The liquid sample is transported through the test strip under the capillary action. The "capillary action" means the well-known physical effect caused by the interaction between the liquid and the wall or inside of the medium, and the physical effect can cause the liquid to flow in the medium. The medium here may be the first absorbent material and the second absorbent material, or may also include the third absorbent material, and of course, it also includes the absorbent material made into the test pad of the test area. It can be understood that the capillary action makes the liquid sample flow based on a fact that these absorbent materials are not moistened because the liquid sample just reaches here. If these dry materials are filled with or absorb the liquid, the liquid will not continue to flow because the capillary action degrades or disappears.

In some embodiments, the test strip further includes a flexible cover that may be an adhesive tape/glue or a mesh; and the test strip can be colored, printed or painted. The cover can roll the test strip, such that the components, such as the reaction pad and the absorbent material, are bonded together. In a specific embodiment, the reaction pad can be viewed through the cover. The "absorbent material" means a substance that is highly absorbent and can transport liquids to other parts by the capillary action. The "third absorbent material" promotes the flow of the liquid as a test sample to the reaction pad, thus promoting the generation of a detectable signal in the reaction pad, and also plays a role in changing the flow direction of the liquid and slowing down the flow rate, such that the liquid sample on the first absorbent material flows slowly to the test pad to isolate the first absorbent material and reduce the direct impact of the liquid sample on the first absorbent material on the test pad.

In different specific embodiments, depending on the property of the test sample or the analytical sample, the sample under test may be a biological sample such as urine or an ecological sample, or may be a food or beverage sample. In various specific embodiments, the test samples may be body fluids, tissues or body fluid derivatives, blood, serum, plasma, saliva, oral fluid, sweat, urine, feces, spinal fluid, vaginal extract, mucus, tissues, milk, wine, food, water and soil. The sample may be a liquid derivative or a solid derivative. "Derived from solid" means that the sample changes from a solid state to a liquefied state. The device of the invention can be used to detect various liquid samples, and these liquid samples may be liquids, solid derivatives, semi-solids mixed with or dissolved in the liquids.

In other specific embodiments, sample analysis includes determining a specific sample property. The "sample property" may be any property of the sample, for example, the presence or absence of the physical property of the analyte or the sample. In different specific embodiments, the analyte may be an oxidant, a reductant, nitrate, nitrite, glutaraldehyde, pyridinium chlorochromate, a heavy metal, a toxic metal, a toxic chemical, blood, a blood component, a blood product, glucose, ketone, peroxidase, protein, creatinine, urobilinogen, bilirubin, bacterium, a bacterial component, and a bacterial product. In other specific embodiments, the physical property includes the pH value, the specific gravity of the sample, and the presence or absence of protein, bacteria, or blood cells.

Another aspect of the invention provides a method for analyzing a liquid sample. The method includes: allowing an end of the device to contact with the liquid sample; allowing a reaction pad to react with the liquid sample for a long enough time to generate a detectable signal; and comparing the signal with a standard signal. In some specific embodiments, the detectable signal may be a change in color on the reaction pad. The "standard" may be any appropriate and objective way of expressing a test result. For example, the standard may be a standard comparison table or card provided for the device, or together with the device, or in other possible ways, and the comparison of the signal with that the standard signal includes the comparison of the color of the reaction pad after the test with the color of the standard table or card, and determining the test results through such comparison. The detectable signal may be indicative of any objective analytical result (for example, fluorescence, enzyme-based assay, or spectrophotometric assay). The standard can be made based on any of these or other testing methods. These methods include: reacting long enough to allow the liquid sample to flow into the test pad along the absorbent strip and react with the reagents on the test pad, and comparing the color of the test pad with that in a standard result comparison table or card at the end of the reaction.

The invention also provides a kit for testing a liquid sample. The kit includes the device of the invention and an operation instruction of the device. In different specific embodiments, the kit can also include an analysis result comparison table or other standards. The kit can be packaged in any suitable form, such as vacuum sealed boxes, plastic bags or aluminum foil bags.

The invention further includes some other useful aspects described in detail here. These aspects can be fully understood by using these products. These aspects need to be further combined with various specific embodiments for investigation to obtain complete evaluation in the invention. In addition, other aspects and specific embodiments of the invention will also be described in detail.

The summary of the invention is not limited to the above description, and other features and benefits of the invention are reflected from the following detailed description and claims.

### BRIEF DESCRIPTION OF THE DRAWINGS

FIG. 1 is a structural schematic diagram according to a specific embodiment of the invention.
FIG. 2A is an illustrative diagram of a liquid flow principle of a conventional lateral flow test strip (a test strip in a conventional prior art).
FIG. 2B is a schematic diagram of a principle according to a specific embodiment of the invention I.
FIG. 2C is a schematic diagram of a principle according to a specific embodiment of the invention II.
FIG. 3 is a structural schematic diagram of a testing element according to a specific embodiment of the invention.
FIG. 4 is a structural schematic diagram of a testing element (with a flexible cover) according to a specific embodiment of the invention.
FIG. 5 is a structural schematic diagram of a testing element according to a specific embodiment of the invention.
FIG. 6 is a structural schematic diagram of a testing element according to a specific embodiment of the invention.
FIG. 7 is a structural schematic diagram of a testing element according to a specific embodiment of the invention.
FIG. 8 is a structural schematic diagram of a testing element according to a specific embodiment of the invention.
FIG. 9 is a structural schematic diagram of a testing element according to a specific embodiment of the invention.
FIG. 10 is a structural schematic diagram of a testing element according to a specific embodiment of the invention.
FIG. 11 is a structural schematic diagram of a testing element according to a specific embodiment of the invention.
FIG. 12 is a schematic diagram showing a specific test result of a testing device according to a specific embodiment of the invention.
FIG. 13 is a schematic diagram showing a specific test result of a testing device according to a specific embodiment of the invention.

### DETAILED DESCRIPTION OF THE INVENTION

In the following detailed description, the accompanying drawings and corresponding textual descriptions are only intended to illustrate particular specific embodiments that are likely to be implemented in the invention. We do not exclude that the invention may also be implemented in other specific embodiments and that the structure of the invention may be altered without violating the application scope of the invention.

### Detection

Detection means to assay or detect presence or absence of a substance or material, including but not limited to, a chemical substance, an organic compound, an inorganic compound, a metabolite, a drug, a drug metabolite, an organic tissue, a metabolite of an organic tissue, a nucleic acid, a protein, or a polymer. In addition, detection means that the amount of a substance or material is tested. Further, assay also means immunoassay, chemical assay, enzyme assay, and the like. In a specific embodiment of the invention, a reaction pad or a test pad is used to test the property of a sample or the presence or absence of an analyte through chemical reaction, and the analyte can also be tested through an immune method.

### Samples

Samples tested by the testing device of the invention include biological fluid (for example, case fluid or clinical sample). Liquid samples or fluid samples may be derived from solid or semi-solid samples, including feces, biological tissues and food samples. The solid or semi-solid specimens may be converted to liquid specimens by any appropriate methods, such as mixing, mashing, macerating, incubating, dissolving, or digesting the solid specimens by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological specimen is urine; and preferably, the biological specimen is saliva. Food samples include food processing substances, final products, meat, cheese, wine, milk, and drinking water. Plant specimens include specimens derived from any plants, plant tissues, plant cell cultures, and media. "Environmental specimens" include specimens derived from the environment (for example, liquid specimens from lakes or other bodies of water, sewage specimens, soil specimens, groundwater, seawater, and waste liquid specimens). The environmental specimens may further include sewage or other waste water.

In some embodiments, the liquid sample is located in the testing chamber, instead of being manually applied to the sample application area of the testing element of the invention, where the sample application area is proximal to the bottom of the testing chamber; when there is the liquid sample at the bottom of the testing chamber, an end portion of the sample application area contacts the liquid sample, and the liquid sample flows to the test pad depending on a capillary force for testing whether it is adulterated; as described above, natural urine has natural properties. However, if the urine is diluted with water, or some additional substances affecting the test affect the pH value, specific gravity or performance of the urine, this means that the urine is not the natural urine but is doped with other substances. Such urine is called "adulterated" urine. This has impact on the specific analyte especially when
small molecules of drugs in the urine are tested. If the urine is adulterated, an invalid test result is made through the test. For example, in a test strip 600, a first end 101 of a first absorbent material and a first end 201 of a second absorbent material overlap with each other and can directly contact the liquid sample. Of course, when such ends are arranged in the testing device, for example, perpendicularly located in the tank of the testing device; the ends are located at a bottom of a urine cup, and the first end 101 of the first absorbent material and the first end 201 of the second absorbent material contact the liquid, such that the liquid can flow on the first absorbent material and the second absorbent material under the capillary force. The first end here may be called the sample application area.

### Downstream and upstream

Downstream or upstream is divided according to a flow direction of a liquid, generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to facilitate the flow of a liquid, a liquid may overcome gravity to flow towards an opposite direction to the gravity; and in this case, downstream or upstream is divided according to a flow direction of the liquid. For example, in the testing device of the invention, when there is the liquid sample at the bottom of the testing chamber, for example, the liquid sample from the sample chamber, such as a urine sample or a saliva sample of a test subject, can flow from an end of the test strip to the other end of the test strip (that is to say, from the bottom to the top).

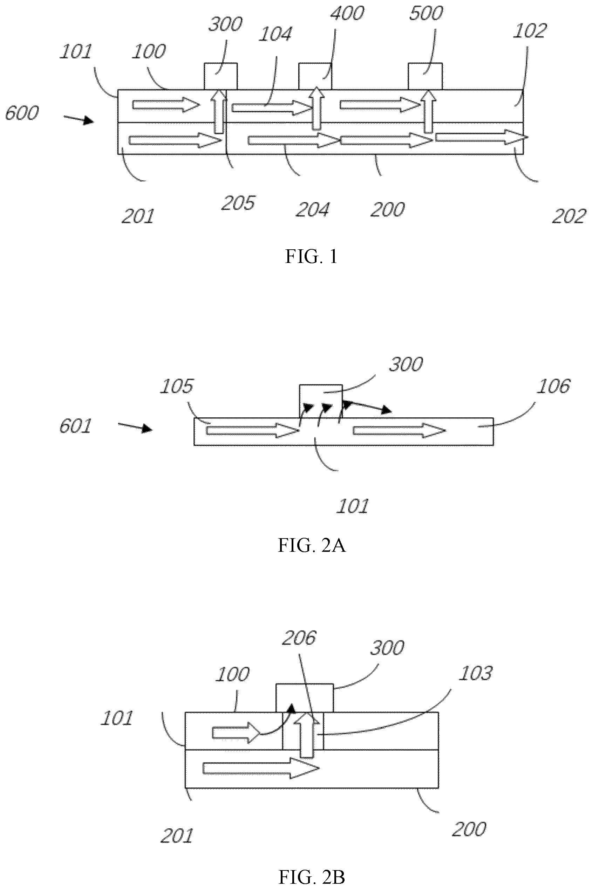

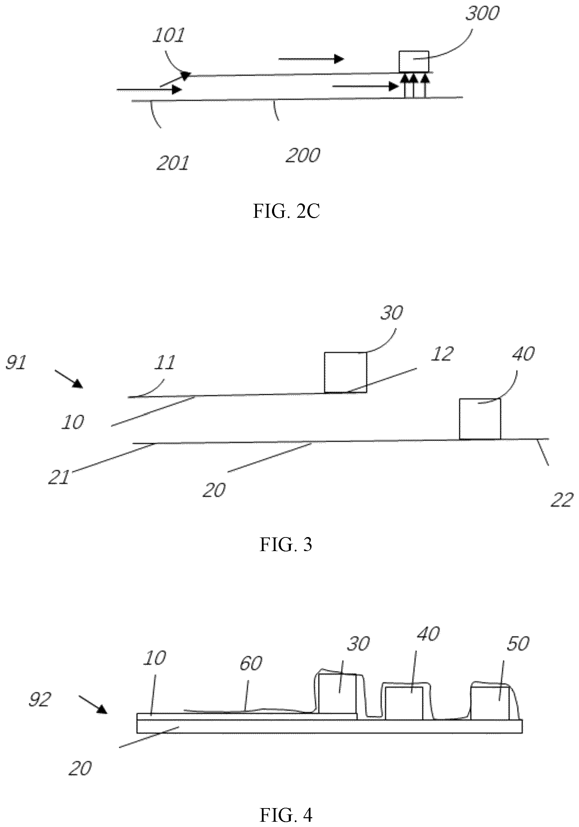

In the invention, according to the flow direction of the liquid, as shown in FIG. 1, a first test pad 300 is located at the upstream of a second test pad and the second test pad 400 is located at the upstream of a third test pad. In this case, the liquid flows from the first ends 101, 201 of the first absorbent material 100 or the second absorbent material 200 to their respective second ends 102, 202. As shown in FIG. 3 - FIG. 5, the liquid flows on different absorbent materials. As shown in FIG. 3, a first test pad 30 is located at the upstream of a second test pad 40, where the upstream involves in the difference in position. The liquid absorbed from the first absorbent material flows from the first end 11 to the second end 12; the second test pad is located on the second absorbent material, and the liquid sample on the second absorbent material flows from the first end to the second end 22. Here, it can be said that the second test pad is located at the downstream of the first test pad. Although they are not located in a same fluid path, they are different in position. Generally, the liquid sample on the second absorbent material flows through the position of the first test pad 30, and then flows to the second test pad 40 located downstream. Of course, the liquid sample from a first absorbent material 10 also flows through the first test pad 30, and then flows to the second test pad 40 located downstream if there is any liquid.

### Liquid communication

Liquid communication means that liquid or gas can flow from one place to another place. In a flow process, the liquid or gas may pass through some physical structures that play a guiding role. The "pass through some physical structures" here means that the liquid passes through the surfaces of these physical structures or their internal space and flows to another place passively or actively, where such passive flow is usually caused by external forces, such as flow under the capillary action and the action of air pressure. The flow here may also be an active flow of the liquid due to self-action (gravity or pressure), and also may be a passive flow therefore. The fluid may be a forward flow or also a reverse flow under the action of air pressure; or the fluid is caused to flow from a position to another position under the action of air pressure. The communication here does not mean that a liquid or a gas is necessarily present, but indicates a relationship or state between two objects under some circumstances. If a liquid is present, it can flow from one object to another. Here it means the state in which two objects are connected. In contrast, if there is no liquid or gas communication state between two objects, and a liquid exists in or on one object but is unable to flow into or on another object, it is a non-communication, non-liquid communication or non-gas communication state.

### Analyte

Examples that can use an analyte related to the invention include some small-molecule substances, including drugs (such as drug of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of drug abuse include cocaine; amphetamine (AMP) (e.g., Black Beauty, white amphetamine tablets, dexamphetamine, dexamphetamine tablets, and Beans); methamphetamine (MET) (crank, meth, crystal and speed); barbiturate (BAR) (such as Valium, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzodiazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The testing device of the invention may also be used to detect drugs belonging to a medical use but easy to be taken excessively, such as tricyclic antidepressants (imipramine or analogues) and acetaminophen. These drugs are metabolized into micromolecular substances after being absorbed by human body. These micromolecular substances exist in blood, urine, saliva, sweat and other body fluids or in some body fluids.

For example, the analyte detected by the invention includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. Chemical analysis of any other clinical urine may be performed by lateral flow test in combination with the device of the invention. The sample of the invention may be urine, and the analyte may be HCG, LH, and other substances, which are used for testing ovulation or early pregnancy.

### Testing element

FIG. 1 shows a lateral flow test strip 600 according to a specific embodiment of the invention. The test strip includes the first absorbent material 100, and a plurality of reaction zones are arranged on the first absorbent material, for example, a first reaction zone with a first reaction pad 300 and a second reaction zone with a second reaction pad 400; and of course, optionally, the first absorbent material may be provided with a third reaction zone on which a third reaction pad or a third test pad 500 can be arranged. Reagents are treated on these test pads or reaction pads, and can be used to contact the liquid sample to test the property of the analyte in the liquid sample or the property of the liquid sample. The above three reaction pads can be used to test the properties of three analytes or three liquid samples, for pH, specific gravity, or nitrite content. Generally, the reaction pad is also made of the absorbent materials that can absorb water. The reaction pad is soaked in a reaction reagent solution, and then dried. The reaction pad contacts the liquid sample to make chemical reaction. Depending on the change in the color of the reaction pad and the color shade of the reaction pad, the concentration of the analyte and the property of the liquid sample can be judged. In one embodiment, the second absorbent material 200 is attached or pasted under the first absorbent material 100, and the reaction area is not directly arranged on the second absorbent material, but the flow rate of the liquid sample on the first absorbent material is slower than the flow rate of the liquid sample on the second absorbent material or the flow rate of the liquid sample on the second absorbent material is faster than the flow rate of the liquid sample on the first absorbent material. Understandably, the flow rate means the liquid flowing distance per unit time, and the long liquid flowing distance per unit time indicates the fast flow rate of the liquid. In one embodiment, the first absorbent material has the first end 101 contacting the liquid sample and the second end 102 opposite to the first end, and a plurality of test pads 300, 400, 500 are arranged on the first end and the second end. The second absorbent material located below the first absorbent material also has a first end 201 and a second end 202 opposite to the first end, and the first end is also used to contact the liquid sample. Of course, when the first end 101 of the first absorbent material and the first end 201 of the second absorbent material contact the liquid sample, the flow rates of the liquids on the two absorbent materials are different, and the flow rate of the liquid sample on the first absorbent material is smaller than the flow rate of the liquid sample on the second absorbent material. FIG. 1 is taken as an example, the first test pad 300 on the first absorbent material is located at a fixed position 205 below which the first absorbent material and the second absorbent material are located; a distance from the first test pad 300 to the first end 101 of the first absorbent material contacting the liquid is the same as a distance from the first test pad 300 to the first end 201 of the second absorbent material contacting the liquid. If the flow rate of the liquid sample on the second absorbent material is fast, the liquid reaches the position 205 below the first test pad 300, but the liquid sample on the first absorbent material does not reach this position 205; in this case, the liquid sample on the second absorbent material is absorbed by the first absorbent material, because a part of an area 103 below the first test pad 300 of the first absorbent material 100 is dry (as shown in FIG. 2B), and after absorbed by the first absorbent material 100, the liquid is absorbed by the first test pad 300 thereon. In fact, it can be understood that the liquid sample absorbed by the first test pad 300 is from the second absorbent material. When the liquid sample on the first absorbent material reaches below the first test pad, the flow rate of the liquid slows down. In a deceleration case, the flow direction of the liquid changes, more liquid is absorbed by the first test pad, and the liquid sample on the first absorbent material will continue to flow downstream 104 at a reduced flow rate; in this case, because the liquid under the first test pad increases (from the first absorbent material and the second absorbent material), the flow rate of the liquid slows down, and the flow direction of the liquid is mainly from the bottom to the top (as indicated by an arrow 206 in FIG. 2B). Therefore, it is not easy to cause the liquid sample on the first test pad to flow back to the first absorbent material, and the chemical reagents on the first test pad to the first absorbent material are not reduced due to the reduction in the reflux. For a fact that the liquid sample on the first absorbent material slows down, one reason is that one area 103 located downstream, for example, the lower part of the first test pad 300 is moistened in advance, and when the subsequent liquid sample on the first absorbent material flows to the area 103 moistened in advance, the flow rate of the liquid naturally decreases; another reason is that the liquid sample on the first absorbent material flows parallel to the test pad, while the liquid sample on the second absorbent material flows perpendicular to the test pad; the perpendicular flow liquid being converged with the parallel flow liquid will change the parallel flow direction of the liquid sample on the first absorbent material, such that the flow rate of the liquid sample on the first absorbent material slows down; with the change in the direction, more liquid will flow to the first test pad 300. More liquid is allowed to flow to the first test pad 300 in a perpendicular direction, thereby overcoming the reflux of the liquid sample on the first test pad. Another reason is that the flow rate of the liquid sample on the first absorbent material to the downstream of the first test pad slows down, and this is also another factor to overcome the reflux of the liquid sample on the first test pad.

As understood herein, the liquid sample on the second absorbent material 200 continues to flow downstream 204, while the flow rate of the liquid sample on the first absorbent material is relatively slow and the liquid sample on the first absorbent material still flows downstream 104, the liquid sample on the second absorbent material flows to the position below the second test pad 400 at the downstream 204, flows through the first absorbent material from the bottom of the second test pad 400 and then is absorbed by the second test pad 400 (as indicated by an arrow). When the liquid sample on the first absorbent material 100 reaches below the second test pad 400, the liquids are converged here, and the liquid absorbed by the second test pad 400 mainly is from the second absorbent material, or a large amount of liquid is from the second absorbent material and a small amount of liquid is from the first absorbent material, such that the change in the flow direction of the liquid can reduce the reflux of the liquid sample on the second test pad 400. In a similar way, when the liquid flows to the end, the liquid sample on the third test pad 500 is from the liquid sample on the second absorbent material, and then from the liquid or liquid sample on the first absorbent material. A method for reducing reflux proposed by the invention is different from a conventional method for reducing reflux. In the conventional method, as described in U.S. Pat. No. US7,595,196, the reflux is reduced by adding a filter pad; and the method features in complicated manufacturing process and increased cost. In the invention, the purpose of accelerating the flow rate here is to hope that the liquid sample on the second absorbent material can reach below the test pad in advance, and then the liquid will flow upward from the second absorbent material below the test pad, and the flow direction of the liquid will be changed. In addition, the flow of the liquid still exists on the first absorbent material, but the flow rate of the liquid will gradually slow down, and the liquid will still moisten the first absorbent material; therefore, this ensures that the test pads 300, 400, 500 can fully absorb liquid samples, and the liquid samples can be absorbed by the test pad. In a case that the first end of the first absorbent material is aligned at the first end of the second absorbent material, the first absorbent material can be a relatively compact absorbent material or a material with a small pore size, such as a compact filter paper, and the second absorbent material can be a porous absorbent material with a relatively large pore size, such that the flow rate of the liquid can be faster. Of course, an external pressure can be applied to the first absorbent material, such that the first absorbent material is compressed, while the second absorbent material remains unchanged, or the flow rate of the liquid can be changed. Of course, a hydrophobic reagent can be added to the first absorbent material, and a hydrophilic reagent can be added to the second absorbent material, so as to increase the fluidity of the liquid. Of course, in more extreme cases, the first absorbent material is substantially made of the non-absorbent material, such as a plastic card, and there are holes in the plastic card. The first test pad 300 covers the holes, and the second absorbent material is located below the plastic card, and there is the absorbent material in the holes; the absorbent material connects to the second absorbent material and the first test pad 300. When the liquid flows to the second absorbent material, part of the liquid sample flows to the first test pad 300 through the absorbent material in the holes. Due to the function of the holes, the liquid can only flow from the absorbent material to the first test pad, and the liquid sample on the test pad will not flow back to the second absorbent material. Although this way is complicated, it is also one way of the invention. Therefore, the so-called "first absorbent material" of the invention has the following meanings: absorbing liquid, where the flow rate of the liquid sample on the first absorbent material is smaller than the flow rate of the liquid sample on the second absorbent material; another meaning thereof is that the first absorbent material does not absorb the liquid at all, but the first test pad, second test pad or third test pad located on the first absorbent material can absorb the liquid sample from the second absorbent material through the through hole. In another embodiment, FIG. 2C is taken as an example, the first end 201 of the second absorbent material is allowed to contact the liquid sample, the liquid sample flows on the second absorbent material, the liquid contacted by the first end 101 of the first absorbent material is from the second absorbent material. In this case, a single liquid flow path is separated into two flow paths at a place where the first end 101 of the first absorbent material contacts the second absorbent material, where a same similar effect can be obtained when the liquid from the flow path on the second absorbent material reaches below the first test pad 300 on the first test area in advance, and the liquid sample on the first absorbent material reaches below the test pad 300 later than the liquid sample on the second absorbent material.

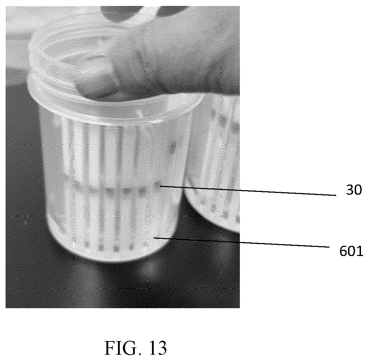

On the contrary, if there is no second absorbent material, the liquid sample only flows on the first absorbent material, for example, as shown in FIG. 2A, when the liquid flows below the first test area, part of the liquid will be absorbed by the test pad 300, and the other part will continue to flow to the test pad 400 located downstream. In this case, during the liquid flow, the liquid sample on the first test pad 300 may flow back to the first absorbent material. The refluxed liquid may contain test reagents or color reagents that have reacted and refluxed to the first absorbent material 100, and it will be found that the downstream 106 of the first absorbent material is stained with colored substances. These substances, such as the colored substances or the test reagents, will flow to the second test pad located downstream with the liquid and then interfere with the reaction of the second test pad, thereby causing incorrect test results. The provision of multiple test pads on a single fluid path has the above defects. On such a conventional test strip, the liquid perpendicularly flows into the test pad 300, but most of the liquid obliquely flows into the test pad. In addition, because the liquid needs to continue to flow downward on the absorbent material, the liquid sample on the test pad 300 may flow back to the absorbent material due to the capillary force at the downstream, which causes the diffusion of the reagent on the test pad 300; or there are the colored substances, and the colored substances will diffuse, such that the color of the test pad 300 is partially lost and appears incomplete, and the area with patches appears, as shown in FIG. 13, where the color is displayed, the conventional test strip 601 is not a complete color, but has patches, that is, the colored substances on the test pad 30 are partially diffused by the downstream liquid and is incomplete.

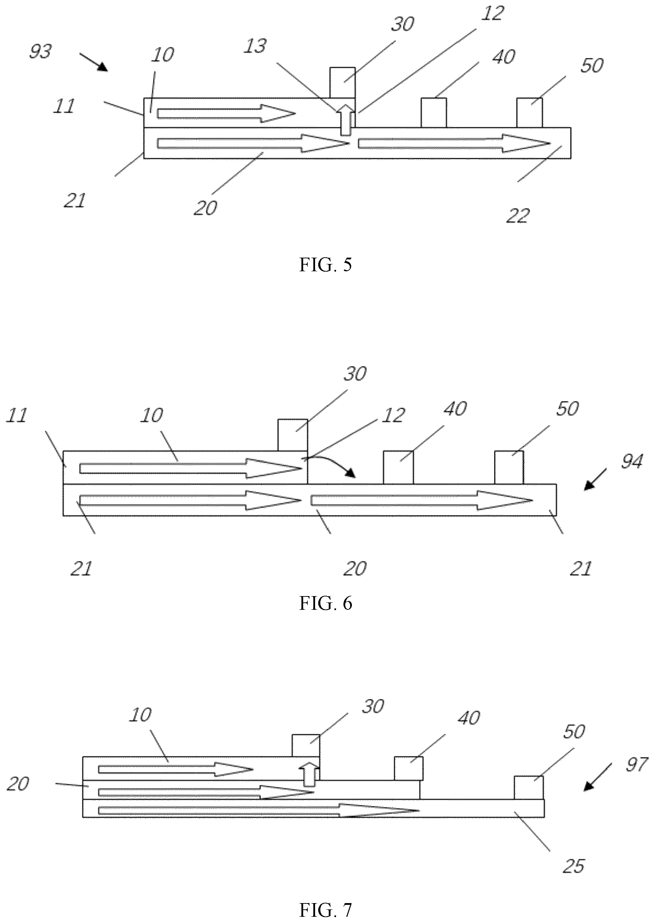

In other embodiments, as shown in FIG. 3 and FIG. 6, the first test area is provided on the first absorbent material 10, a first test pad 30 is provided on the first test area, and a second test pad 40 is provided on the second absorbent material and located at the downstream of the first test pad. In this case, the first absorbent material and the second absorbent material may make the liquid sample thereon have a different or same flow rate. When the liquids have the same flow rate, because the fluid path of the first absorbent material is relatively short and the fluid path of the second absorbent material is relatively long; relatively speaking, a distanced from the first test pad 30 to the first end 11 of the first absorbent material contacting the liquid is smaller than a distance from the second test pad 40 to the first end 21 of the second absorbent material contacting the liquid; therefore, when the first ends of the first absorbent material and the second absorbent material contact the liquid, the liquid flows on the two materials. After the liquid sample on the first absorbent material is absorbed by the first test pad, the liquid will not continue to flow (the capillary force disappears or decreases), while the liquid sample on the second test area on the second absorbent material is mainly from the liquid sample on the second absorbent material. Therefore, it is equivalent to two fluid paths, and the liquid in each fluid path is provided to one test pad separately; and the fluid paths are staggered. The liquid sample on the first absorbent material is absorbed by the first test pad, and even if the liquid continues to flow forward, the capillary action on the liquid thereon is reduced (relative to the second absorbent material) because the length of the first absorbent material is longer than that of the second absorbent material, the liquid also flows on the second absorbent material and continues to flow downstream under the great capillary action, such that the liquid of the first absorbent material rarely or hardly flows to the second absorbent material. Therefore, the interference to the second test pad 40 is reduced. After all, it is difficult for the liquid flowing back from the first test pad to flow to the second absorbent material, thus preventing the chemical reagent on the first test pad 30 from flowing to the second test pad 40.

In a preferred embodiment, the first test pad 30 is located at the end 12 of the first absorbent material. When the liquid flows to the end 12 of the first absorbent material, the liquid will not continue to flow along the first absorbent material, and no liquid will continue to flow downstream on the first absorbent material, thus naturally reducing the driving force to drive chemical reagents on the first test pad 30 and avoiding the reflux of the liquid sample on the first test pad 30. For example, as shown in FIG. 2A, the liquid sample on the absorbent material needs to continue to flow downstream due to the presence of the capillary action, causing the reflux of the liquid sample on the test pad. However, as shown in FIG. 3, there is only one test pad 30 on the first absorbent material or on the end; the driving force for the liquid to continue to flow is reduced or the capillary force disappears, so the reflux of the liquid sample on the test pad 30 is overcome, the diffusion of reagents on the test pad is naturally avoided, and the impact on the second test pad 40 located downstream and on the second absorbent material is also avoided. According to the design here, another absorbent material 25 can be provided, and a third test pad 50 is arranged on the absorbent material. The relatively remote liquid has three relatively independent fluid paths, and each fluid path is provided with one test pad (as shown in FIG. 7).

In some embodiments, as shown in FIG. 4, the first absorbent material 10 and the second absorbent material 20 are combined together, the first test pad 30 is located on the first absorbent material and especially on the end, and the second test pad 40 and the third test pad 50 are sequentially arranged on the second absorbent material. A transparent membrane covers the first absorbent material and serves as the cover 60. The transparent membrane is used to fix the test pad and protect the test pad from being contaminated; in addition, the test results on the test pad can be observed through the transparent membrane.

In some embodiments, for example, as shown in FIG. 5 and FIG. 7, a preferred embodiment is that the flow rate of the liquid sample on the second absorbent material 20 is greater than the flow rate of the liquid sample on the first absorbent material 10, test pads are independently arranged on each absorbent material, for example, the first test pad 30 is arranged on the first absorbent material 10, and the second test pad 40 is arranged at the downstream of the second absorbent material 20 located at the downstream of the first test pad 30. As shown in FIG. 5 and FIG. 7, due to the fast flow rate of the liquid sample on the second absorbent material, the liquid reaches the bottom 13 of the first test pad 30 in advance (the first test pad is dry and has the capillary force). Because the liquid sample on the first absorbent material does not reach the first test pad, the liquid will flow from the second absorbent material to the first test pad 30 in a vertical direction through the bottom 13 of the first test pad, and when the first absorbent material reaches the end 12, the liquid will continue to be absorbed by the first test pad. However, the liquid will not continue to flow along the first absorbent material 10 (the capillary force of the first absorbent material itself is used up and there is no capillary force to allow the liquid to continue to flow), thus reducing the reflux or reverse flow of the liquid sample on the first test pad 30 to the first absorbent material. As shown in FIG. 6, in case of the same flow rate of the liquid sample on the two test strips, although the reagent flowing from the first test pad 30 to the second absorbent material can be reduced and the interference to the second test pad located downstream can be reduced, it is still possible that the reagent will flow to the second absorbent material, and the flow rate is the same, and the time to reach the first test pad 30 is the same. In this case, although the first absorbent material itself has no capillary action, the second absorbent material still has capillary force; generally, the end 12 of the first absorbent material contacts with and is combined with the second absorbent material. If the flow rate is different, the problem of diffusion can be solved better, but there is still a chance of diffusion. Although the flow direction of the liquid sample on the first absorbent material changes and the flow rate of the liquid decreases, the liquid sample on the first test pad 30 may flow back to the second test pad because the second liquid material still has the capillary force. Of course, different flow rates can better solve the problem of diffusion than the same flow rate, and the probability of diffusion is much smaller.

Of course, in the above way, the another absorbent material 25 is arranged below the second absorbent material, and the third test pad 50 (as shown in FIG. 7) is arranged on the absorbent material, such that the flow rate of the liquid sample on the another absorbent material 25 can be greater than the flow rate of the liquid sample on the second absorbent material 20, and of course, they all contact the liquid sample at the same time.

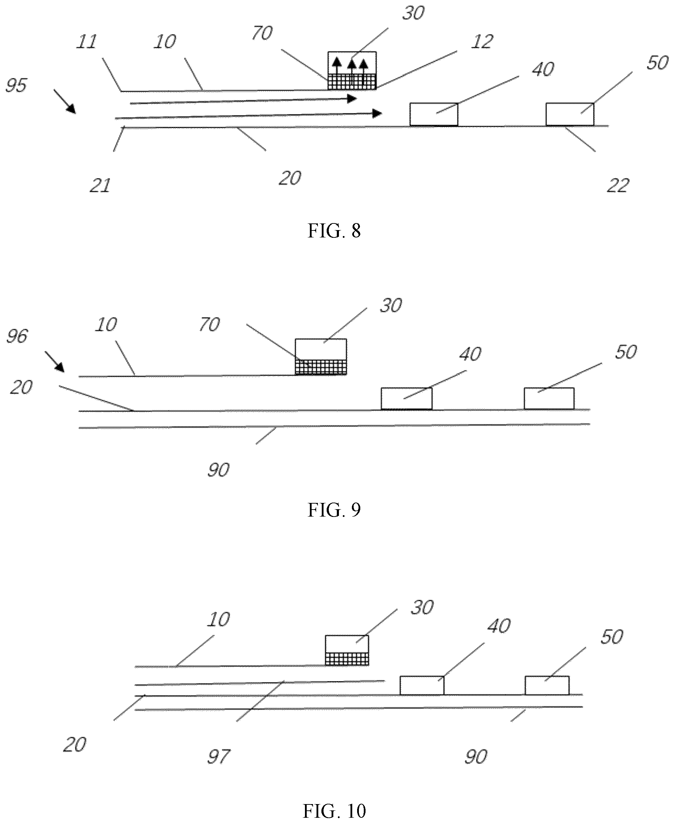

Therefore, in some embodiments, as shown in FIG. 8, a third absorbent material 70 is arranged between the first test pad 30 and the first absorbent material, and connects to the first absorbent material and the first test pad. This way is a preferred technical way of the invention. The third absorbent material has a property of slowing down the flow rate of the liquid flowing rapidly from the first absorbent material 10, and the flow rate is mainly the flow rate of the liquid flowing rapidly into the first test pad. In fact, it can be understood that the first test pad 30 is also made of the absorbent material, and needs to absorb the liquid from the first absorbent material. However, during the liquid absorption, it is not desirable for the liquid to flow back. The time for the liquid to flow back includes the reaction time after the test pad absorbs the liquid and contacts the liquid (if the liquid sample includes an analyte or has an inherent property, such as pH value), and the reaction will generally change color. The change in color is generally caused by the appearance or accumulation of the colored substances on the test pad. If the reflux of the liquid is avoided, the first test pad can better accomplish the reaction independently, and an adverse impact on the test pad located downstream (for example, the second test pad) is not caused. Therefore, different test pads can be independently tested to ensure the accuracy of the test. In the invention, the first test pad 30 is arranged on the first absorbent material 10, and the second test pad 40 is arranged on the longer second absorbent material 20 located at the downstream of the first test pad; this relatively solves the problem of reflux, but does not completely solve it.

This is due to the fact that, under time requirements for rapid diagnosis, the test strip of the invention is often used together with other test strips. For example, in the drug test, the other test strips are used to test whether a specific drug molecule exists in the liquid sample by an immunization method, while the test strip of the invention is mainly used to test whether the liquid is adulterated, for example, water, strong acid and alkali or other irrelevant substances are added into the liquid. The purpose of adulteration is to interfere with the immunoassay and causes the results to be inconsistent with the actual results. Therefore, one purpose of the test strip of the invention is to test whether the liquid sample is adulterated, and it is always hoped that the result will appear for the first time. If the result indicates that the liquid is not adulterated, this means that other immune test results are reliable. If the liquid is adulterated, this means that other immune test results are unreliable, there is no point in continuing the test, and samples need to be collected again for another test. Therefore, in practice, it is always hoped that the results of adulteration will be obtained as soon as possible, meaning that the results on the first test pad 30 or other test pads 40, 50 will appear as soon as possible; therefore, it is inevitable that the first absorbent material 10 or the second absorbent material 20 will have the relatively fast flow rate of the liquid, and it is also hoped that the liquid is sufficient, such that the test pad can contact the liquid quickly and the reagents on the test pad can contact the liquid quickly, so as to obtain the test results as soon as possible. In a case that the liquid flows quickly, the possibility of the reflux of the liquid sample on the test pad will be inevitably caused. The third absorbent material 70 is arranged between the test pad 30 and the first absorbent material 10, which can better solve the above problems. In addition to the function of diverting the liquid, the third absorbent material 70 has the function of changing the flow direction of the liquid and slowing down the flow rate of the liquid. For example, as shown in FIG. 8, when the first absorbent material 10 contacts the liquid sample, a large amount of liquid is absorbed by the first absorbent material, flows rapidly to the end, and is accumulated at the end; although the driving for the liquid to continue to flow is reduced at the end, in practice, the liquid sample on the second absorbent material 20 still flows under capillary action. Therefore, if the flow rate of the liquid sample on the second absorbent material is greater than the flow rate of the liquid sample on the first absorbent material; when the liquid sample on the first absorbent material reaches the end, the liquid sample on the second absorbent material 20 has flowed downstream of the first test pad 30; therefore, even if the liquid sample on the first absorbent material is redundant and the second absorbent material has been moistened, this also reduce the driving force for the liquid sample on the first absorbent material to flow or continue to flow downstream. In addition, as mentioned above, the liquid flowing through the first absorbent material 10 has a fast flow rate, and a large amount of liquid is accumulated at the end. If the third absorbent material is arranged between the end surface of the first absorbent material and the test pad 30, the flow rate of the liquid can slow down, and the flow direction of the liquid also changes, and most of the liquid flows slowly to the test pad 30 through the third absorbent material 70. It can be understood that the test pad is gradually moistened from the bottom of the test pad to the upper surface thereof. The flow direction of the liquid is perpendicular to the direction of the test pad 30, such that the direction of the liquid changes and the flow rate of the liquid slows down, and the reflux of the liquid sample on the test pad 30 is avoided to the greatest extent. In the conventional design, as shown in FIG. 2A, in addition to providing the liquid for the first test pad, the absorbent material for transporting the liquid under the test pad needs to continue to move rapidly downstream to provide the liquid for the test pad located downstream, such as the second test pad. The direction of the liquid will not change substantially. The liquid does not perpendicularly moisten the test pad, but obliquely flows into the test pad. The liquid has the flow rate and the acceleration of movement (the movement of the liquid is similar to the movement of an object, and the liquid will continue to move forward when the driving force disappears suddenly). Therefore, the liquid flowing to the test pad will flow back, and the liquid will continue to flow downstream on the absorbent material, such that the chemical reagent on the test pad flows to the absorbent pad 100 and pollutes other test strips located downstream (this is a form of diffusion). In some embodiments, in order to achieve the function of the third absorbent material 70, the water absorption capability needs to be considered. In one embodiment, it is best not to choose a material with the same properties as the first absorbent material, and preferably, for example, to choose a material with a tight texture and a relatively slow flow rate of a liquid, where the slow flow rate of the liquid is relative to the flow rate of the liquid sample on the first absorbent material. To speed up the flow rate of the liquid, generally, the liquid with a large pore size and capillary and a loose texture quickly flows. On the contrary, the liquid with a small pore size and a tight texture, such as the absorbent material with large mass per unit volume, flows slowly. For example, the first absorbent material is polyamide fiber, and the thickness of the membrane is 0.6-1.0 mm. The absorbent material has natural water absorption property, and the polyamide fiber membrane with an area of 60 mm × 10 mm can absorb 0.6 gm, +/-0.15 gm of liquid. Such an amide fiber membrane can be purchased from Filtrona FibertecTM (Colonial Heights, VA). When the absorbent materials are polyester fibers, the first absorbent material may be light in weight per unit volume, the third absorbent material may be heavy in weight per unit volume, and the second absorbent material may be lighter in weight per unit volume than the first absorbent material. These are all freely chosen and combined by persons skilled in the art through limited experiments. Of course, it can be understood that the first absorbent material, the second absorbent material and the third absorbent material have a same thickness, so naturally there are dense materials with a slow flow rate and loose materials with a fast flow rate. In some embodiments, the test strip further includes a support structure 90, and the support structure is to allow the test strip to have certain rigidity, for example, a support sheet can be a plastic sheet. The fast and slow flow rates of the invention are relative, and the selection of such materials is easy for the persons skilled in the art; the fast and slow flow rates can be compared by a conventional test method; the absorbent materials with same specifications (namely, a same length and a same thickness) are provided, and their ends contact the liquid. In a unit time (minutes or seconds), the long movement distance of the liquid indicates the fast flow rate of the liquid.

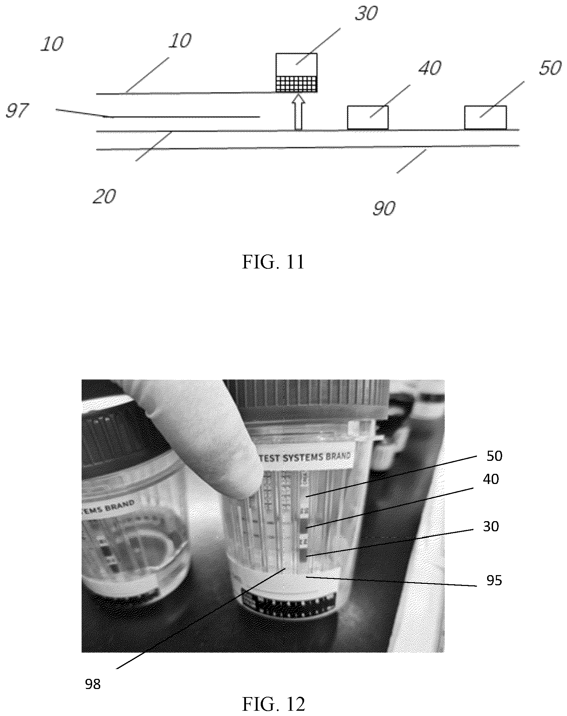

In some embodiments, in order to prevent the fluid paths between the first absorbent material and the second absorbent material from interfering with each other, an impermeable membrane 97 can be arranged between the first absorbent material and the second absorbent material, and actually completely isolates the exchange of the liquid between the first absorbent material and the second absorbent material. For example, as shown in FIG. 10, the impermeable membrane covers the second absorbent material 20 and extends to the downstream of the end 20 of the first absorbent material. Therefore, the end 12 of the first absorbent material 20 does not directly contact with the second absorbent material, and the liquid sample at the end of the first absorbent material will not flow onto the second absorbent material due to the capillary force provided by the second absorbent material. The impermeable membrane can be coated with a glue on both sides thereof, such that the first absorbent material and the second absorbent material can be bonded to one side of the impermeable membrane respectively, and the first test pad 30 is arranged on the other side of the first absorbent material, and the second test pad 40, 50 is arranged at a place where the second test pad is not covered by the impermeable membrane 97. In some embodiments, the impermeable membrane is located between the first absorbent material and the second absorbent material, but does not exceed the end of the first absorbent material (as shown in FIG. 11); and the impermeable membrane is absent from the area 108 of the test pad 30 with one end contacting the liquid. Therefore, when the flow rate of the liquid sample on the second absorbent material is greater than the flow rate of the liquid sample on the first absorbent material, the liquid can directly flow through the area 108 and then onto the test pad through part of the first absorbent material.

In some embodiments, a hard backing 90 is arranged below the first absorbent material 20, is relatively hard material and can play a supporting role, such that the whole test strip has rigidity and can be assembled conveniently, for example, the test strip can be assembled in a test card or a test tank.

Therefore, the absorbent material of the invention can be composed of any substance that supports capillary flow, including but not limited to polyamide fiber, polyester, filter paper, nitrocellulose, water absorption meshes, glass meshes that becomes absorbent after treatment, and the like. Absorbent materials 20, 30, 70 can freely transport liquid samples from one end of the test strip to the other end thereof within the time required for effective determination by the capillary action. This usually takes only a few minutes.

Many different absorbent materials can be used in the invention. Any substance that can effectively transport liquid by means of the capillary action of the liquid can be used as the absorbent material. In a specific embodiment, the first absorbent material is a polyamide fiber, and the thickness of the membrane is 0.6-1.0 mm. The absorbent material has natural water absorption property, and the polyamide fiber membrane with an area of 60 mm × 10 mm can absorb 0.6 gm, +/-0.15 gm of liquid. Such an amide fiber membrane can be purchased from Filtrona FibertecTM (Colonial Heights, VA). Of course, other absorbent materials can also be used in the invention. For example, surfactants that are often widely used as filter materials and contain amine or carboxylic acid groups (as substrates for binding various substances) can also be used in the invention. These materials are cut into strips and can be used as good absorbent materials; in addition, the materials can be purchased from Filtrona FibertecTM (ColonialHeights, VA). In other specific embodiments, the absorbent material includes but is not limited to cotton fiber. Polyester pretreated with detergents, a protein and a buffer can also be used as a useful absorbent material in the invention.

In the invention, the first absorbent material and the second absorbent material have one contact end 101, 11, 201 contacting the liquid sample, and the liquid sample is absorbed into the absorbent material when the contact end is inserted into the liquid sample. In a specific embodiment, the test strip of the invention has only one contact end 101, 11, 201, that is, the liquid sample is introduced into the absorbent material through the contact end of the absorbent material. According to different testing requirements, some special devices include a plurality of test strips. The absorbent material is usually rectangular or strip-shaped, or may also be other shapes without affecting the test results. In a specific embodiment, the test strip of the invention includes at least two reaction pads for testing different properties of samples. In other specific embodiments, the test strip includes at least 3, at least 4, at least 5, at least 6, or at least 7 reaction pads. In some embodiments, the test strip is generally rectangular, and the reaction pads are equidistantly arranged along the absorbent material. These reaction pads are not arranged on the same absorbent material, but on different absorbent materials, for example, the first test pad or the reaction pad is arranged on the first absorbent material, and two test pads 400, 500, 40, 50 are arranged on the second absorbent material. Of course, it can be understood that two test pads can be arranged on the first absorbent material, and other two pads can be arranged on the second absorbent material and located at the downstream of the first absorbent material. A design principle of such arrangement is that the substances on the test pad on the first absorbent material will not interfere with the reaction of the test pad located downstream, or the test pads with slight impact on the reaction of the test pad located downstream will be arranged upstream in advance, while the test pads that interfere with the reaction will be arranged downstream or finally tested.

The reaction pads or test pads 300, 400, 500, 30, 40, 50 include reagents required for detection and can provide detectable signals related to the properties of the samples. In those specific embodiments, the reaction pads are chemical test pads, and these chemical test pads are very useful in the invention. They include reaction pads of test strips in a form of "dipping samples - reading results", especially reaction pads saturated with chemical indicators, where the reaction pads can generate detectable signals according to the properties of the samples when used for analysis and detection. The following table summarizes some test pads currently available.

| Physical properties/test items | Test items | Possible marks |
|---|---|---|
| pH | pH | Sample contamination or adulteration |
| | | Disease conditions |
| Specific gravity | Specific gravity | Sample dilution and disease conditions |
| Bleacher, peroxide, and the like | Oxidant | Sample adulteration |
| Creatinine | Creatinine | Sample dilution and disease conditions |
| Nitrite and nitrate | Nitrite/nitrate | Sample adulteration and disease conditions |
| Glutaraldehyde | Glutaraldehyde | Sample adulteration |
| Protein (specificity or non-specificity) | Protein | Disease conditions |
| Blood, and hemameba | Blood/ and hemameba | Disease conditions |
| Sugar | Sugar | Disease conditions/diabetes |
| Heavy metals, toxins, and the like | Heavy metals, toxins, and the like | Sample or environmental contamination, intoxication |
| Ascorbic acid | Vitamin C | Other test inhibitors (namely, protein, blood sugar, and the like); commonly used for dietary test |
| *Escherichia coli 0157: H7, S. aureus, Salmonella,* C. *perfringens, Campylobacter, L. monocytogenes, V. parahaemolyticus, B. cereus* | Bacteria contamination | Food or beverage contamination |
| Tumor marker | Tumor marker | Disease conditions |

According to different use intentions of the test strip of the invention, a plurality of different reaction pads can be arranged on different absorbent materials, and these absorbent materials overlap with each other to form the test strip, or the invention can be realized in different forms and specific embodiments. For example, when the invention is used to test drugs or metabolites of drugs in urine, whether subjects adulterate the urine to cover up the fact of drug abuse should be confirmed for the first time. In this case, the test strip can be equipped with pH, specific gravity, oxidants, and nitrite indicators. The test of these items can reveal the presence or absence of adulteration, because known specific gravity and general adulteration will show suspicious test results on one or more reaction pads, such that further investigation and confirmation can be made. The reaction pad is composed of a 3MM filter paper, and other kinds of filter papers and absorbent materials can also be used.

In other specific embodiments, obstetricians can test the pH, specific gravity, protein, sugar and/or other specific metabolic markers in urine of pregnant women through a urine analysis test strip to find metabolism or other pregnancy problems. During physical examination, the test strip having the function of testing the pH, specific gravity, protein and blood reaction pad can also be used to determine whether the bladder of a patient is infected. In another case, a health inspector can use the test strip to test whether beef is contaminated by Escherichia coli 0157:H7 or Salmonella. With reference to the invention, other fields that can be associated by those skilled in the art and reaction pad combinations that can provide important information in these fields all fall within the scope of the invention.

The cover 60 can protect the test strip. In a specific embodiment, the cover 60 is adhesive and can be used to roll together various constituents of the test strip, such as absorbent materials and reaction pads. The cover 60 can be made of any suitable material as long as the reaction pad can be observed through the cover. In some specific embodiments, the cover 60 is a transparent adhesive tape. In another specific embodiment, the cover 60 is a transparent mesh. In different specific embodiments, the mesh can be made of nylon, polyester, fiber and other materials suitable for the cover. The cover 60 can be applied to the test strip by any suitable manufacturing method (such as coloring or painting on the test strip). For example, the test strip can be protected by spraying a clear waterproof paint or an ink onto the test strip and the components of the test strip can be rolled together.

In other specific embodiments of the invention, the support 90 is used to provide a backing for the test strip, and can be made of any suitable material, such as plastic, non-absorbent paper or cardboard, glass, metal or aluminum foil. The support as a bottom board is arranged below the absorbent material.

When the liquid sample flows into the reaction pads 300, 400, 500, 30, 40, 50, the reagents in the reaction pads react with the analytes in the samples and generate detectable signals showing the test results. These reagents can also react with the liquid samples to determine the properties of the liquid samples (such as pH, specific gravity, or other physical properties). The "reagent" refers to a chemical substance that reacts with the liquid sample and can produce the detectable signal showing the test results. The detectable signal can be compared with the standard signal. In a specific embodiment, the detectable signal indicates the change in color on the reaction pad, and the test result is determined by comparing such color with the color in the standard table or card. The standard table or card indicates positive and/or negative test results by the color, or provides quantitative evaluation associated with the test results. The determination of pH is taken as an example. In a specific embodiment, the reaction pad is beige at the beginning (for example) and turns yellow when reacting with the liquid sample, and the result shows that the pH value is low (acidic). If (for example) the reaction pad reacts with the liquid sample and turns blue, the result shows that the pH value is high (alkaline). The color produced on the reaction pad is between yellow and blue, such as yellow-green or blue-green, which indicates that the result is neutral.

### Using method

The invention also provides a method for using the device. For example, the first end 101, 11 of the first absorbent material of the test strip 600, 601, 91, 92, 93, 94, 97, 95, 96 and the first end 201, 21 of the second absorbent material thereof contact the liquid sample; and the test strip may stay in the sample for a period of time, such as about 1-10 minutes, such that a sufficient amount of the liquid sample flows into the reaction pad. The liquid sample flows from the first end 101 to the second end 102 under the capillary action, and then flows into the reaction pad to react with the reagent on the reaction pad. After the reaction, the detectable signal (such as the change in color) on the reaction pad is compared with the standard signal standard (such as that in a standard color comparison table). The comparison can be done by any suitable method, such as visual inspection or using an electronic detector or a scanner.

In another specific embodiment, the test strips 600, 91, 92, 93, 94, 95, 96 can be combined with the testing device, such as a lateral flow analysis urine cup. For example, the test strip can be combined with a single or multiple drug test strips or plates to help technicians judge whether the subjects try to cover up drug abuse and adulterate urine samples.

In another specific embodiment, the test strip 600, 91, 92, 93, 94, 95, 96 can be used in a doctor ward in combination with the urine cup. In this case, the technicians do not need to contact the urine sample directly. The urine can be tested and analyzed when flowing to the urine cup ("0 step" cup), or the technicians can start some devices of the urine cup for test and analysis ("1 step" cup or "2 step" cup). As shown in FIG. 12, any test strip can be inserted into a plastic tank, for example, the test strip 95 is inserted into a carrier tank, the water absorption end 101, 201 is allowed to be arranged at the bottom of the urine cup, and the properties of the urine, such as specific gravity and pH value, are evaluated by chemical methods. However, the other test strip 98 is used to test the analytes in the urine by the immune method, such as small molecular substances of drugs. During the test, the immunoassay test strip is used to test whether there are the small molecules of drugs in the urine, while the test strip 95 is used to test whether the urine is adulterated depending on the colors or the change in the colors displayed on the test pad 30, 40, 50 or whether other substances exist.

### Kit

The invention also provides a kit including one or more test strips 100. The kit includes a test strip, a standard comparison table (such as a color table that displays test results based on a change in color), and may further include an operation instruction of the device. As a part of a device serving as saliva test and the like, the test strip 100 can be provided in various ways, such as a small tank including a plurality of test strips, various urine cups or sample cups.

### Embodiment 1-1: Configuration of test strip (Embodiment 1 of the invention)

Before the first absorbent material is selected, polyamide fiber absorbent materials with different specifications can be selected to determine the flow rate, that is, a test strip with a same size (with a same length, thickness and width) is provided; one end of the test strip is inserted into the liquid to observe the moistening distances of the fiber absorbent materials with different specifications in a unit time, where the unit time may be minutes or seconds; and the moistening distances are sorted from long to short, and the long moistening distance indicates the fast flow rate. The first absorbent material and the second absorbent material may have the same length, but the flow rate of the liquid sample on the second absorbent material is greater than the flow rate of the liquid sample on the first absorbent material; for example, as shown in FIG. 1, the first absorbent material and the second absorbent material have the same length, the time when the liquid sample on the second absorbent material flows from the first end 201 to the first test pad 300 may be 30-60 seconds, and the time when the liquid sample on the first absorbent material flows from the first end to the first test pad 30 may be 50 seconds or 100 seconds; and the distance from the first test pad 300 to the first end of the first absorbent material is the same as the distance from the first test pad 300 to the first end of the second absorbent material. The materials are combined according to the order of the flow rate from fast to low. The thickness of each absorbent material is about 0.1-0.2 mm, but the flow rate of the liquid is different.

The test strip is composed of a thin sheet including multiple test strips, and then cut into a single test strip.

The complete sheet includes a 10.16 cm × 27.94 cm strip-shaped pressure-sensitive adhesive backing paper (support sheet) with an adhesive surface. The adhesive surface can be exposed by tearing off a protective paper. A piece of polyamide fiber absorbent material (Filtrona FibertecTM, second absorbent material) with a same size is placed above the adhesive backing paper, and then the first absorbent material (polyamide fiber) with the same size is placed on the second absorbent material, and such fiber absorbent materials have different flow rates (the flow rate of the liquid sample on the first absorbent material is 80s/5cm, and the flow rate of the liquid sample on the second absorbent material is 60s/5cm). Three narrow water-soluble adhesive strips (AS-110 medical acrylic adhesive) with a size of about 0.2 cm × 27.9 cm are arranged on the first absorbent material at an interval of 0.5-0.8 cm, and 3MM reaction pad test strips with a size of 0.5 cm×27.9 cm are arranged thereon and located on the surface of the first polyamide fiber absorbent material. Before the reaction pad is pasted, the reaction pad test strips are pretreated with reagents for detecting and analyzing subject matters. One reaction pad is used for testing creatinine, another reaction pad is used for testing nitrite, and the last reaction pad is used for testing glutaraldehyde. After the reaction pad is pasted, the paper is covered with a pressure-sensitive transparent adhesive membrane and transversely cut into individual test strips (about 0.5 cm wide), where each test strip includes creatinine, nitrite, and glutaraldehyde reaction pads (similar to those in FIG. 1).

Embodiment 1-2: The test strip with the same specification is manufactured by the same method, but it lacks the second absorbent material and only includes the first absorbent material, and the others are the same as those in Embodiment 1, and the structure of the test strip is similar to that of the test strip as shown in FIG. 3A.

Embodiment 1-3: The complete sheet includes a 10.16 cm × 27.94 cm strip-shaped pressure-sensitive adhesive backing paper with an adhesive surface. The adhesive surface can be exposed by tearing off a protective paper. Then, a layer of the second absorbent material (polyamide fiber) with the same size covers the adhesive surface; and a layer of the first absorbent material (polyamide fiber) covers the second absorbent material. The width of the first absorbent material is 3 cm and the length thereof is 27.9 cm (long sides of the first absorbent material and the second first absorbent material are aligned). A narrow water-soluble adhesive strip (AS-110 medical acrylic adhesive) with a size of about 0.2 cm × 27.9 cm is arranged at the center of the first absorbent material adjacent to the thin sheet, and then a 3MM reaction pad test strip with a size of 0.5 cm × 27.9 cm is arranged thereon for testing creatinine. A narrow water-soluble adhesive strip (AS-110 medical acrylic adhesive) with a size of about 0.2 cm ×27.9 cm is arranged on the second absorbent material distanced from the test pad for testing creatinine about 1 cm; and a 3MM reaction pad test strip with a size of 0.5 cm × 27.9 cm is further arranged thereon for testing nitrite. Then, a narrow water-soluble adhesive strip (AS-110 medical acrylic adhesive) with a size of about 0.2 cm × 27.9 cm is arranged at a location distanced from the test pad for testing nitrite about 2 cm in width, and a 3MM reaction pad test strip with a size of 0.5 cm×27.9 cm is arranged thereon for testing glutaraldehyde. In the embodiment, the flow rate of the liquid sample on the first absorbent material is the same as the flow rate of the liquid sample on the second absorbent material (similar to the structure of the test strip as shown in FIG. 3 and FIG. 6), where the flow rate is 80s/5cm. The first end 11 of the first absorbent material and the first end 21 of the second absorbent material are allowed to contact with the liquid through the test strip.

Embodiment 1-4: the test strip in Embodiment 1-4 is the same as that in Embodiment 1-3, but the flow rate of the liquid sample on the second absorbent material is greater than flow rate of the liquid sample on the first absorbent material. The time when the liquid sample on the second absorbent material is allowed to flow from the first end 201 to the first test pad 300 can be 60s per 5 cm, and the time when the liquid sample on the first absorbent material is allowed to flow from the first end to the first test pad 30 can be 80s per 5 cm; the distance from the first test pad 300 to the first end is the same as the distance from the first test pad 30 to the first end; and the structure of the obtained test strip is similar to that of the test strip as shown in FIG. 5.

Embodiment 1-5: the structure of the test strip in Embodiment 1-5 is similar to that in FIG. 8 or FIG. 9, other structures are the same as those in Embodiment 1-4, but an absorbent material 70 more compact than the first absorbent material is arranged below the test pad and between the first absorbent materials, and the flow rate of the liquid sample on the absorbent material 70 is slower and needs to be 100s/5cm. The width of the test strip is the same as the width of the test pad.

### Embodiment 2 - Determination of urine adulteration

A total of 5 ml solution is prepared by the following method. Analysis of creatinine in urine: urine with creatinine at an original concentration of 140 mg/d1 is diluted with deionized water to have creatinine concentrations of 10 mg/dl, 20 mg/d1, 50 mg/dl, and 100 mg/dl.

Analysis of nitrite in urine: nitrite is respectively added to 4 portions of 5 ml nitrite-free urine to obtain their respective final concentrations of 0 mg/dl, 10 mg/dl, 30 mg/d1, and 80 mg/dl.

Analysis of glutaraldehyde in urine: glutaraldehyde is added to glutaraldehyde-free urine to obtain their respective concentrations of 50 mg/d1, 150 mg/d1, 1000 mg/dl and 2000 mg/dl.

Test strips are prepared as described in Embodiments 1-1 to 1-5. The assays are conducted independently for three days, and five test strips are used for each adulteration concentration. Thus, each adulteration concentration (creatinine, nitrite, or glutaraldehyde) is tested a total of 45 times. The test strip is inserted into the sample for 1-2 seconds and placed on a paper towel to remove the redundant liquid sample; after 1-3 minutes, the color of the test strip is compared with that in a standard color table and the test results are read; whether the test pad has an intact color and whether the downstream of the test pad tends to diffuse are observed; and the observations are recorded.

A creatinine reaction pad changes from yellow to brown in the presence of creatinine. According to known concentrations, whether correct results can be obtained through test is determined and whether brown substances diffuse to the downstream of the first absorbent material is observed. Through the observation, it is found that in Embodiment 1-2, the test strips present serious diffusion, where about 25 test strips diffuse downstream of the first absorbent material, and some of the test strips diffuse to the test pad located downstream and used for testing nitrite with the liquid (a diffusion rate is about 55%). For the diffusion observation of Embodiment 1-1, about 15 test strips diffuse (the diffusion rate is about 33%), for the diffusion observation of Embodiment 1-3, about 10 test strips diffuse (the diffusion rate is about 22%), for the diffusion observation of Embodiment 1-4, 3 test strips diffuse (the diffusion rate is about 6.6%), and for the diffusion observation of Embodiment 1-5, no diffusion occurs downstream. This shows that, compared with the conventional test strips, in different embodiments of the invention, the diffusion (which actually indicates the reflux of the liquid) of colored substances of the test pad is alleviated, and the optimal embodiment is Embodiment 1-5 in which almost no diffusion phenomenon occurs.

Glutaraldehyde is not a routine component of urine. If it is detected, this means that the urine may be adulterated. A glutaraldehyde reaction pad changes from rose red to purple in the presence of glutaraldehyde. According to known concentrations, the correct results can be obtained from each reaction pad. The test results for glutaraldehyde concentrations of 0-50 mg/dl can be clearly distinguished through visual inspection of the reaction pad before and after the change in color. Statistically, the test strips in Embodiment 1-2 present serious diffusion, where about 22 test strips diffuse; for the diffusion observation of Embodiment 1-1, about 12 test strips diffuse; for the diffusion observation of Embodiment 1-3, about 6 test strips diffuse; for the diffusion observation of Embodiment 1-4, 5 test strips diffuse; and for the diffusion observation of Embodiment 1-5, no diffusion occurs.

Similarly, the diffusion phenomenon of a nitrite test pad is observed and presents a similar rule. The test strips in Embodiment 1-2 present serious diffusion, where 30 test strips diffuse, while the test strips in Embodiment 1-5 of the invention have no diffusion.

### Embodiment 3 - Drug screening for employment

The embodiment illustrates the application of the invention in drug screening before employment. Employers require test strips that can detect drug abuse. "Drug of Abuse" refers to the use of a drug (typically functions to paralyze the nerves) not directed to a medical purpose. Abuse of these drugs will lead to physical and mental damage, dependency and addiction (substantially). Examples of abuse of drug include cocaine; amphetamine (AMP) (e.g., Black Beauty, white amphetamine tablets, dexamphetamine, dexamphetamine tablets, and Beans); methamphetamine (MET) (methamphetamine, meth, stimulant and amphetamine); barbiturate (BAR); lysergic acid diethylamine (LSD); sedatives; pain relievers (such as selective serotonin uptake inhibitors), phencyclidine (PCP); tetrahydrocannabinol (THC); and opiates (such as morphine, opium, codeine and heroin). In order to achieve the purpose of the test, it is necessary to conduct a urine adulteration test. For example, because creatinine and protein assays are included in the test strip, the test strip can test the dilution of the urine sample, as well as B vitamins, glutaraldehyde, nitrite, chromate, acetic acid, VisineTM (Pfizer Co. Ltd., New York), sodium bicarbonate, DranoTM (S.C. John, Racine, Wisconsin), soft drinks, oxidants such as bleacher, hydrogen peroxide, pyridine, pyridinium chlorochromate, or other chemical substances added to the urine in an attempt to adulterate. The invention can be used in any suitable form, such as the urine cup (as shown in FIG. 12).

Prospective employees are sent to the testing office for a urine test. If the urine cup including the test strip is used for testing, the subjects collect a urine sample into the urine cup and hand it over to a tester to be tested according to the specific requirements of the device used. The tester records the test results and feeds them back to the employers. Excess samples can be discarded, or kept for later use, or sent to the result confirmatory laboratory.

The embodiment describes a device for testing contamination of well water as a source of drinking water and a using method thereof. Drinking water samples taken from the well are placed in a suitable container. In this case, items tested by the test strip include pesticides, ozone, atrazine, arsenic, trihalomethane, polychorinated biphenyls (PCBs), radon, organic phosphate, methyl tributyl ether (MTBE), nitrate, lead, iron and manganese, as well as biological substances such as Escherichia coli, Giardia and Crytosporidium. Any chemical or biological substances to be analyzed can be combined for testing in the device. Water samples are collected from wells under test, the test strips are placed in water containers, the water samples are determined according to the instructions of the device used, and after a few minutes, the color of the reaction pad is compared with that in the standard table, and the presence or absence of contaminants or the level of contamination is determined through comparison. If the test results show that the well includes any substances that should not be there, the water source should be replaced until the contamination is eliminated.

### Embodiment 4-food monitoring

The embodiment describes a device for testing contamination of food and a using method thereof. All kinds of food-borne pathogens have been concerned by FDA and local health departments. Raw materials of meat, dairy products and other food products usually need to be tested for contamination by Escherichia coli and Salmonella. In such detection, lead, mercury, botulinum toxin, pesticides, chloramphenicol, listeria, other bacteria such as Bacillus cereus, Campylobacter, Clostridium, Staphylococcus, Vibrio and Yersinia should also be tested. These tests can be conducted reliably and cheaply by using the invention. The testing device used in the embodiment includes the test strip for testing the reaction pad of Escherichia coli and Salmonella. Monitoring personnel take a small amount of samples from the beef under test, mix such samples with the buffer in a test tube, insert the test strip into the test tube, wait for 3-5 minutes to make the buffer flow to the second end of the test strip under the capillary action, compare the color of the reaction pad with that in a standard table in a test strip package, record the test results, and continue relevant tests or report the results.

All the patents and publications mentioned in the description of the invention indicate that these are public technologies in the art and can be used by the invention. All the patents and publications cited herein are listed in the references, just as each publication is specifically referenced separately. The invention described herein can be realized in the absence of any one element or multiple elements, one restriction or multiple restrictions, where such restriction is not specifically described here. For example, the terms "comprising", "essentially consisting of' and "consisting of" in each example herein may be replaced by the rest 2 terms. The term "alan" herein merely means "one", but does not exclude including 2 or more instead of including only one. The terms and expressions which have been employed herein are descriptive rather than restrictive, and there is no intention to suggest that these terms and expressions in this description exclude any equivalents, but it is to be understood that any appropriate changes or modifications can be made within the scope of the invention and appended claims. It can be understood that the embodiments described in the invention are some preferred embodiments and features. Any person of ordinary skill in the art can make some modifications and changes according to the spirit of the description of the invention. These modifications and changes are also considered to fall within the scope of the invention and the scope limited by independent claims and dependent claims.

## Claims

1. A testing device, comprising a first absorbent material for contacting a liquid sample, wherein a first test area is arranged on the first absorbent material; and a second absorbent material for contacting the liquid sample, wherein a second test area is arranged on the second absorbent material.

2. The testing device according to claim 1, wherein the liquid sample absorbed by the first absorbent material is substantially absorbed by the first test area, and the liquid sample absorbed by the second absorbent material is substantially absorbed by the second test area.

3. The testing device according to any one of claims 1-2, wherein the first absorbent material has a first end contacting the liquid sample and a second end opposite to the first end, and the second absorbent material has a first end contacting the liquid sample and a second end opposite to the first end, wherein the first end of the first absorbent material is aligned at or overlapped with the first end of the second absorbent material.

4. The testing device according to any one of claims 1-3, wherein the first test area is located at the second end of the first absorbent material.

5. The testing device according to any one of claims 1-4, wherein the first test area is located at the upstream of the second test area.

6. The testing device according to any one of claims 1-5, wherein the first test area comprises a first test pad, the second test area comprises a second test pad, and the first test pad or the second test pad comprises a reagent thereon, wherein the reagent is capable to contact an analyte in the liquid sample to indicate the absence or presence or content of the analyte.

7. The testing device according to any one of claim 1-6, wherein a length of the first absorbent material is smaller than a length of the second absorbent material.

8. The testing device according to any one of claim 1-7, wherein a third absorbent material is arranged between the first test area and the first absorbent material, and the liquid sample on the first absorbent material flows onto the first test area through the third absorbent material.

9. The testing device according to claim 8, wherein the water absorption capability of the third absorbent material is lower than the water absorption capability of the first absorbent material.

10. The testing device according to any one of claim 8-9, wherein a flow rate of the liquid sample on the first absorbent material is allowed to slow down through the third absorbent material, such that the liquid sample flows into the first test area.

11. The testing device according to any one of claims 8-10, wherein a flow rate of the liquid sample on the third absorbent material is smaller than the flow rate of the liquid sample on the first absorbent material.

12. The testing device according to any one of claim 8-11, wherein a density per unit volume of the third absorbent material is greater than a density per unit volume of the first absorbent material.

13. The testing device according to any one of claims 1-12, wherein a flow rate of the liquid sample on the first absorbent material is smaller than a flow rate of the liquid sample on the second absorbent material.

14. The testing device according to any one of claim 1-13, wherein when the first absorbent material and the second absorbent material contact the liquid sample at the same time, a time when the liquid sample is absorbed to the first test area through the second absorbent material is earlier than a time when the liquid sample is absorbed to the first test area through the first absorbent material.

15. The testing device according to any one of claims 6-14, wherein the analyte comprises PH, nitrous acid, hemoglobin or urea.
